(19)

Europäisches
Patentamt
European
Patent Office
Office européen
des brevets

(11) **EP 3 332 778 A1**

(12) **EUROPEAN PATENT APPLICATION**
published in accordance with Art. 153(4) EPC

(43) Date of publication:
**13.06.2018 Bulletin 2018/24**

(21) Application number: **16844787.8**

(22) Date of filing: **01.08.2016**

(51) Int Cl.:
***A61K 31/00*** *(2006.01)*  ***A61K 31/4045*** *(2006.01)*
***A61K 31/57*** *(2006.01)*  ***A61P 15/00*** *(2006.01)*

(86) International application number:
**PCT/RU2016/000498**

(87) International publication number:
**WO 2017/044003 (16.03.2017 Gazette 2017/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.08.2015 RU 2015132315**

(71) Applicant: **Obshchestvo S Ogranichennoj Otvetstvennost'yu "Nauchno-Vnedrencheskij Centr Agrovetzashchita"**
**129329 Moscow (RU)**

(72) Inventors:
• **ENGASHEV, Sergey Vladimirovich**
**Moscow 127221 (RU)**
• **GERMANOVA, Mariya Sergeevna**
**Kaluzhskaya obl. 249032 (RU)**

• **GERMANOV, Sergey Borisovich**
**Novokuznetsk 654084 (RU)**
• **ENGASHEVA, Ekaterina Sergeevna**
**Moscow**
**127282 (RU)**
• **NOVIKOV, Denis Dmitrievich**
**303142 (RU)**
• **GLADUSHKO, Yana Yur'evna**
**Lomonosov**
**188512 (RU)**
• **KHOMISHIN, Dmitriy Vladimirovich**
**Naro-fominskiy raion**
**Moskovskaya obl. 143333 (RU)**
• **GERMANOVA, Ol'ga Leonidovna**
**Novokuznetsk 654084 (RU)**
• **MUROMTSEV, Aleksandr Borisovich**
**Kaliningrad 236029 (RU)**

(74) Representative: **Lapienis, Juozas**
**MSP Europe UAB**
**21-92 Seimyniskiu Str.**
**09236 Vilnius (LT)**

(54) **METHOD FOR CONTROLLING SEXUAL BEHAVIOUR IN MALE MAMMALS**

(57)    The invention relates to the veterinary field. The method for temporarily blocking sexual functions in male mammals, including behavioural phenomena (seeking a partner, vocalization, etc.) is provided. The method includes single/double administration to an animal of pharmaceutical composition comprising $\alpha$-adrenergic blocking agent tropic to the posterior nuclei of the hypothalamus, melatonin and steroid compound with gestagen activity as active substances. The method provides the opportunity, while using the minimal dose of the steroid hormone, to inhibit sexual behaviour of male animals for the period of several weeks to half a year (depending on the daytime length). The pharmaceutical composition is intended for the transdermal administration during any period of cycle. The use of the drug is not accompanied by side effects, characteristic for this type of medicines.

EP 3 332 778 A1

**Description**

**Field of the Invention**

The invention relates to the veterinary field in particular to a method for temporarily blocking or suppressing sexual functions in male mammals, including behavioural phenomena (seeking a partner, dromomania, intraspecific aggression and aggression towards the host, territorial "marking", vocalization etc.). The method includes single/double administration to an animal of pharmaceutical composition comprising α-adrenergic blocking agent tropic to the posterior nuclei of the hypothalamus, melatonin and steroid compound with gestagen activity as active substances. The method provides the opportunity, while using the minimal dose of the steroid hormone, harmful to health of animals to inhibit sexual behaviour of males for the period of several weeks to half a year. A pharmaceutical composition is administered transdermally in the "Spot-on" form.

**Background of the Invention**

Sexual behaviour of male dogs and cats in spring period (extending daytime period to long daytime period) is activated, which leads to unpleasant for the owners of animals changes in their behaviour. Males become aggressive (including often and towards the host), restless, actively search for a partner, which is accompanied by dromomania - "escape", in which case animals are often lost, the probability of infection with zoonotic diseases, including rabies, rise sharply. Hardly pleasant are also behavioristic characteristics of males, such as territorial "marking", vocalization, animals can scratch and gnaw home furnishings etc. Therefore the problem of suppressing primarily the behavioral phenomena of sexual behaviour of males in the veterinary medicine of small domestic animals is extremely relevant. The most effective method for solving these problems is sterilization of male animals, especially since surgical intervention in the case of males is simpler, safer and cheaper than in the case of females. However, in some cases irreversible castration of males may be unacceptable, for example, when using valuable animals for breeding, for exhibition and service dogs, etc. Castration significantly changes the behaviour of the animal, gives the features of apathy and passivity, substantially affects the metabolism and the exterior of animals (in particular the tendency to obesity, progression of diabetes, vomiting, arterial hypertension, thrombophlebitis, thromboembolism, gynecomastia sharply increases). Thus, there is a need for the pharmaceutical management of the sexual functions of males, not accompanied by irreversible changes in the organs and systems of an animal. In humane medicine, the use of temporary chemical castration (both women and men) is known by the administration of increased amounts of hypothalamic releasing hormones in the form of depots (prolonged injectable forms), which leads to disruption in the synchronization of the hypothalamus-hypophysis system, and thereby temporarily blocking gonadotropins produced by the hypophysis from entering the blood (Shchekina E.G. Muzhskaia kontratseptsiia - zavtrashnii den'! [Men's contraception - future!]. Provisor, 2004, ed. 11). Mostly this method is used in oncology for the treatment of hormone-dependent tumors (Mashkovskij M.D. Lekarstvennye sredstva [Medicines]. 16-th edition, revised, amended and supplemented. Moscow, Novaja volna, 2012). To use this method in the veterinary medicine of small domestic animals, the development of convenient injectable forms with slower release and adequate doses is necessary. The use is also limited by the high cost of both the releasing hormones themselves and the components of the prolonged injectable forms. Most widespread was the administration of large doses of gestagens to males, similar to females, which eventually inhibits the production of gonadotropins by the feedback mechanism and causes the effects of temporary chemical castration. In Russia, the same drugs based on megestrol acetate are widely used as applied for contraception of females [RU2163809, published on 10.03.2001]. However, in order to develop a reliable and rapid suppression of sexual behaviour in males, it is necessary to administer gestagens for a longer period and(or) at higher doses, because the response of pituitary structures in males is lower due to the specificity of the receptors. The use of gestagens to influence the sexual behaviour of males is accompanied by a large number of side effects, often irreversible: the adrenal cortex is depressed and the ejection of the corticosteroids is disrupted, pancreas is often damaged with development to varying degrees of diabetes, hepatotoxic effects are almost always detected, cholecystitis occurs. Especially male cats are characterized by the progression of infections of the urogenital system, with the manifestation of cystitis and pyelonephritis and subsequent urolithiasis (bladder stone disease). Acromegaly is often diagnosed in young males. The hyperplasia of mammary glands with frequent occurrence of adenocarcinoma at a later stage is almost always diagnosed. Behaviour of animals becomes passive, they gain excessive weight. The functioning of the gonads is disrupted to a great extent and for a long time, which leads to unsuccessful matings after a long time after the termination of the administration of gestagens. The slow development of the effects of sexual behaviour suppression often does not save from "escaping" in the wake of female being in the state of estrus, with all the consequences. Thus, attempts to influence the sexual behaviour of males only by administering of gestagens demand better solutions.

It is known to use for the purpose of contraception in women the combination of gestagens and melatonin with possible additional administration of estrogen [RU2114621, published on 10.06.1998], as well as the combination of gestagens and more active melatonin derivatives [EP0641565, published on 08.03.1995]. Melatonin is a hormone of the pineal gland, normalizes biological rhythms, has an adaptogenic effect on the body, is a relatively harmless substance and is used in a wide therapeutic range and a wide range of doses. So, for example, at therapeutic doses it is used to improve male and female gametogenesis [EP2253228, published on 24.11.2010]. However, melatonin at higher doses suppresses ovulation, and in combination with steroids it allows to reduce the dose of the latter in the drug, which neutralizes their side effect (Hundanov L., Gladkov A., Levashova V. Zhit' dolgo i schastlivo. Uchenyh zainteresoval melatonin [Live long and happy. Scientists interested in melatonin]. Medical newsletter, 1997, no. 10 (77), pp. 1-3.). For the purposes of contraception of sheep, goats and other breeding animals melatonin and its derivatives in the form of an implant are used for both females (in order to delay estrus) and for males (reducing sperm production) [EP0246910, published on 25.11.1987]. The disadvantage of this invention is the complex process of manufacturing the implant, as well as the difficulties for domestic animals owners, consisting in administration thereof subcutaneously to the restless pets.

For the purpose of contraception and to reduce the sexual activity of both males and females in veterinary medicine a combination of $\alpha$-adrenergic blocking agents with gestagens and(or) estrogens is known (Korhov V.V. et al. Postkoital'nye steroidy i nesteroidnye kontraceptivy [Postcoital steroids and nonsteroidal contraceptives]. Pharmacology and toxicology, 1980, vol. 43 (1), pp. 94-96; Korhov V.V., Veshchilova T.P. Kontraceptivnaja aktivnost' sochetanija steroidov i antiadrenergicheskih preparatov [Contraceptive activity of a combination of steroids and antiadrenergic drugs]. Bull. experim. biol. and med., 1975, vol. 79 (4), pp. 77-79; RU2233586, published on 10.08.2004). The disadvantage of this invention is the low contraceptive effect of the combination (55%) and inaccessibility in the market of the synthetic gestagen used.

The object of the provided invention is to find the most safe and convenient for use method for temporarily blocking sexual functions in male mammals, including behavioural phenomena (seeking a partner, vocalization, etc.). Technical result of the provided invention consists in obtaining of high-efficiency pharmaceutical composition for "Spot-on" applying during any period of sexual cycle, which helps easily solve the problems of change in sexual behaviour of male mammals easily.

### Disclosure of the invention

When studying contraceptive effects of the combining melatonin and gestagens in order to obtain at least the effect of suppressing aggressive reactions in "sleeping around" males, we have additionally introduced known non-selective $\alpha$-adrenergic blocking agent, proroxan (Mashkovskij M.D. Lekarstvennye sredstva [Medicines]. 16-th edition, revised, amended and supplemented. Moscow, Novaja volna, 2012), which interacts with alpha-adrenoreceptors of the posterior nuclei of the hypothalamus responsible for the formation of sexual behaviour. Proroxan reduces rut, suppresses aggressiveness, and normalizes vestibular function without manifestation of significant sedative and other side effects. Surprisingly, it was found that in such case, the correction of the active sexual behaviour of male animals occurs after one/two applications of the above-described combination of biologically active substances and lasts from several weeks to half a year. The effect develops rapidly (from 12 hours), with blocking behavioural phenomena of sexual behaviour - seeking a partner, vocalization, aggressiveness, rut etc., and juvenile features manifest obviously - activation of play and investigative behaviour, the animal becomes friendly. We have not identified this combination of active substances for reducing active sexual behaviour in the literature.

Administration of gestagen alone, or administration of gestagen in combination only with melatonin or only with $\alpha$-adrenergic blocking agent, as well as administration of melatonin only with $\alpha$-adrenergic blocking agent, has not allowed to obtain significant efficacy of the composition and to reduce as much as possible the dose of the steroid component in the drug.

As gestagen we have chosen cyproterone acetate, which has a high affinity for androgen receptors in both the hypothalamic and pituitary structures in males and blocks them without showing androgenic effect that prevents rapid arresting of sexual activity. Replacement of the commonly used megestrol acetate by cyproterone acetate allowed to significantly increase the effect, reduce the loading with progestogenic hormone and increase the physiological effect on the body of males. Thus, the efficacy of this effect is higher when the drug is administered during the evening time, which makes the hypothesis about the prevalent melatonin significancy in the development of the effect legitimate. Introduction of $\alpha$-adrenergic blocking agent to the composition made it possible to suppress the symptoms of the restless behaviour of males as fast and prolongably as possible.

As a non-selective $\alpha$-adrenergic blocking agent it is possible to use proroxan or(and) butyroxan (see examples 16, 17, 18 and other) with nearly identical by properties, or biologically active substances (BAS) with similar by pharmacological and chemical properties. Other tested selective and non-selective $\alpha$-adrenergic blocking agents were not so effective and did not cause the described effect (see example 31), which is apparently caused by the specific influence of proroxan and butyroxan on brain structures.

As the progestin component, gestagens with the highest affinity for the corresponding receptors are used: 6-chloro-1$\beta$,2$\beta$-dihydro-17-hydroxy-3'H-cyclopropa[1,2]pregna-1,4,6-triene-3,20-dione acetate (cyproterone acetate), or(and) 17α-cyanomethyl-17β-hydroxyestra-4,9-diene-3-one (dienogest), or(and) (6R,7R,8R,9S,10R,13S,14S,15S,16S,17S)-1,3',4',6,6a,7,8,9,10,11,12,13,14,15,15a,16-hexadecahydro-10,13-dimethylspiro-[17H-dicyclopropa-[6,7:15,16]cyclopenta[a]phenanthrene-17,2'(5'H)-furan]-3,5'(2H)dione (drospirenone). The use of cyproterone acetate is most relevant because of the commercial availability of the hormone.

For gestagen and α-adrenergic blocking agent the doses should not exceed the commonly used therapeutic ones, because otherwise the likelihood of adverse side effects increases significantly. Melatonin is practically safe at a very significant overdosing.

One might expect that melatonin and proroxan as a part of transdermal forms will have a good transcutaneous bioavailability, and gestagen, which concentrations in the animal's blood are not so critical for the intensity of the effect, also with a certain formulation of the transdermal pharmaceutical composition, will entry in sufficient quantity into the bloodstream and will have a systemic action with minimal local effects. A pharmaceutical composition intended for transdermal administration has been selected, containing (weight%):

| | |
|---|---|
| Gestagen | Therapeutic amounts |
| Melatonin | Therapeutic amounts |
| α-Adrenergic blocking agent tropic to the posterior nuclei of the hypothalamus | Therapeutic amounts |
| N-Methyl-N-(1,2R,3R,4R,5S-pentahydroxyhexyl)-octadecanamide (Stearylamide meglumine) | 0.5 |
| D-Panthenol | 0.5 |
| Polyoxyethylated α-tocopherol | 9.3-10.2 |
| Polyoxyethylated lanolin | 1 |
| 3-(Octadecyloxy)-1,2-propanediol (Batilol) | 0.1 |
| Carboxylic acid | 0.1-1.1 |
| N,N-Diethyltoluamide | 13.9-15.3 |
| 1,2-Isopropylidene glycerol | 18.6-20.4 |
| 1,2,3-Triacetoxypropane , | up to 100 |

which provided for a biological effect at "Spot-on" administration similar to oral administration methods; use of this drug is, however, more convenient. No skin irritation is detected, animals are unaware of the administration process and do not feel any discomfort in the area of application, thus there is no scratching or licking.

The best results have been observed with the combination of the following components (weight%):

| | |
|---|---|
| Cyproterone acetate | 0.9-2.0 |
| Melatonin | 10-17.2 |
| Proroxan base | 0.9-2.0 |
| N-Methyl-N-(1,2R,3R,4R,5S-pentahydroxyhexyl)-octadecanamide | 0.5 |
| D-Panthenol | 0.5 |
| Polyoxyethylated α-tocopherol | 10 |
| Polyoxyethylated lanolin | 1 |
| 3-(Octadecyloxy)-1,2-propanediol | 0.1 |
| Stearic acid | 1.1 |
| N,N-Diethyltoluamide | 15 |
| 1,2-Isopropylidene glycerol | 20 |
| 1,2,3-Triacetoxypropane | up to 100 |

Based on the minimal amounts of BAS lower concentrations of biologically active substances are selected, resulting in an assured effect with a technically admissible amount of the ready-to-use "Spot-on" pharmaceutical form for a given animal species (see examples 9, 12, 19). Thus, for male cats the optimal single volume dose is 0.5-2 ml, as in case of a larger amount solution will spread over an intolerably large area and dampen the hair. The upper concentration limits for proroxan and cyproterone acetate are selected based on the known toxicity data for these components at convenient amounts of "Spot-on" applied form and their solubility in the pharmaceutical composition (see examples 20, 24). The upper concentration limit for melatonin is defined by its solubility in the selected solvent composition (see example 22).

The necessity for pH stabilization of the composition at the level of 4-6 was also found important, as the higher pH means faster BAS destruction (for instance, cyproterone acetate), and the lower pH means the likelihood of skin irritation in the animal (see examples 32, 33). This pH value is easily obtained by adding additive with ionometric control using corresponding carboxylic acid: glycyrrhizinic, or(and) succinic, or(and) lactic, or(and) pyruvic, or(and) citric, or(and) malic, or(and) tartaric, or(and) stearic, or(and) myristic, or(and) octanoic, or(and) lauric acids (see examples 16, 18, 21, etc.) etc. Practice has shown that the desired amount of carboxylic acid varies depending on the molecular weight and dissociation degree from 0.1 to 1.1 weight%.

The biological tests carried out (see examples 6-15) demonstrated, that the developed pharmaceutical composition when applied once (more rarely twice) to males rapidly and completely blocks behavioural phenomena of an active sexual behaviour, with the effect which develops for 12-72 hours and usually maintains from one month to half a year, which allows to minimize the amount of the steroid compound administered to the animal's organism, and therefore the likelihood of grievous and often lethal side effects, developing during long-term systemic administration of hormonally active steroids to the animal. Juvenile characteristics of the animal's behaviour - play and investigative behaviour without aggressiveness - observed after application of the drug, are beneficial for the owners of animals. The drug does not affect the skills of service dogs, the inhibition of sexual behaviour favourably affects the productivity of their work. The drug reversibly blocks fertile functions of males, which are restored immediately after the cessation of its action. The pharmaceutical composition is convenient in use, it is used for transdermal administration as "Spot-on" form during any period of sexual cycle. Melatonin present in the composition according to the literature data (Katzung B.G. Bazisnaia i klinicheskaia farmakologiia [Basic and Clinical Pharmacology]. Vol. 2. Moscow, Binom, 2008, 784 p.) enhances emotional background, has strong antioxidant and adaptogenic effect, and normalizes sleep.

Thus, a safe and convenient method for temporarily blocking the sexual functions of mammalian males, including behavioural phenomena, has been provided, and a pharmaceutical composition for its implementation has been found.

**Embodiments**

Example 1. (Acute toxicity study in white mice)

Materials and methods

An acute toxicity study of the drug prepared according to the example 16 was carried out for apparently healthy male white mice weighing of 23-25 g previously kept in the 15-day-long quarantine. Statistical groups consisted of 6 individuals. In total, 5 groups were formed. The animals were observed for two weeks after the administration of the drug, reporting about the terms of death, the general condition of the animals, the maintaining of motor functions, appetite, the state of the coat, respiration, and reaction to external stimuli.

Tests were carried out in accordance with the "Methodological Recommendations for the Study of the General Toxic Effects of Pharmacological Substances" approved by the Ministry of Health of Russia, 2005.

The experimental drug was administered to white mice intragastrically through an atraumatic metal probe at increasing doses according to Litchfield-Wilcoxon: 2.0 g/kg, 4.0 g/kg, 6.0 g/kg, 10.0 g/kg and 12.0 g/kg. Control animals were fed water in the same volumes. The results are shown in Table 1.

Table 1

| The results of the acute toxicity study of the experimental drug with intragastric administration (mice) | | | | | |
|---|---|---|---|---|---|
| Dose, g/kg | 2.0 | 4.0 | 6.0 | 10.0 | 12.0 |
| Survived | 6 | 5 | 3 | 2 | 0 |
| Died | 0 | 1 | 3 | 4 | 6 |

$LD_{50}$ was 7.2 g/kg (calculated according to the Kerber's formula) with a standard error of 0.97 (according to Gaddam's formula).

Using graphical method of "dose-effect" relationship analysis, the $LD_{16}$ and $LD_{84}$ were determined as 3.48 g/kg and 11.5 g/kg correspondingly.

Results

It was found that $LD_{50}$ (intragastr.) = 7.2$\pm$0.97 g/kg, which allows to classify this drug as 4 class of hazard (low toxicity compound) in accordance with GOST 12.1.007-76.

The acute poisoning clinical aspect was characterized by severe distress, torpidity of animals almost immediately after administration. Then narcosis came, from which part of the animals restored, and another part died on the 2-3 days. When performing pathoanatomical autopsy in dead animals, there were observed hyperaemia of the gastric mucosa, haemorrhages on the course of blood vessels, and plethora of the liver and kidneys.

Example 2. (Study of absorption through skin of the provided drug)

Materials and methods

Study of absorption through skin of the provided drug prepared according to the example 18 was performed in single and repeated (5 days) experiments on white male rats weighing 180-200 g. For the study apparently healthy individuals without visible damage of the skin cover were selected. The animals were divided into three groups:

1st group - control (10 animals);

2nd group - single treatment (10 animals);

3rd group - five-fold treatment (10 animals).

The purpose of the single experiment was determination of lethal doses and determination of cutaneous-oral coefficient (Coef. c/o) correspondingly:

$$\text{Coef. c/o} = \frac{LD50skin}{LD50stomach}$$

For this purpose drug at the volume of 1 ml (5.0 g/kg) was applied to the clipped skin area 4x4 in size on the rat's back (2 times during two hours). Total dose was 10 g/kg.

Results

The clinical findings of intoxication of animals after a single treatment was practically no different from the control. The animals were active, lively and were well fed. Animal death was not reported. Similar results were obtained in a five-fold experiment, where the drug was applied daily at dose of 5.0 g/kg (1.0 ml per rat).

Skin-irritating effect of the drug was not reported in any of the experiments. No erythema of the skin, scratches, cutaneous dropsy or skin fold thickening were observed.

Conclusion: thus obtained data show that the toxic compounds of the drug scarcely absorbed through skin.

Example 3. (Study of the tolerance of provided drug in male dogs)

Materials and methods

Study regarding tolerance of the drug prepared according to the example 16 was conducted in 6 apparently healthy half-breed male dogs at the age of 15 months to 4 years and weighing 3.5-4.7 kg. The animals were arranged into 3 groups, 2 individuals in each group:

1st group - each animal was treated with 1 ml of the drug;

2nd group - each animal was treated with 3 ml of the drug;

3rd group - control (without treating).

Drug was applied onto intact skin along the spinal cord between the scapulas within 10 days with break at the 3 and 6 days. Individuals in the control group were treated with distilled water. Clinical observations were performed a day before application of the drug and after 1, 2 and 5 days after the first application. Examination of the animals included measurement of body temperature, measuring of heart rate and respiratory movements, visual assessment of general condition and determination of the haematological values.

Results

Multiple application of the drug at the therapeutic dose as well as at the dose three times greater than the therapeutic dose did not cause any irregularities in terms of the physiological condition of the animals.

The body temperature ranged from 38.0 to 38.5°C (with the physiological normal range of 37.5-39.0°C). The heart rate was within the range of 70-77 heart beating per minute (the normal range is 70-80). The rate of respiratory movements ranged from 15 to 30 (the normal range is 10-30). The general condition of male dogs in the experimental groups was not different from that of the control animals. Depression and food refusal of individuals in the second group were noted during the last 2 days of the experiment. The results of the haematological study are presented in Table 2.

Table 2

| № | Group | Erythrocytes, $10^{12}$/l | Hemoglobin, g/l | Leucocytes, $10^9$/l |
|---|---|---|---|---|
| Effect of the experimental drug on the morphological blood composition of male dogs | | | | |
| 1 | Treatment with the drug at the therapeutic dose | 8.0±0.1 | 169.3±0.4 | 11.5±0.3 |
| 2 | Treatment with the drug at the dose three times greater than the therapeutic dose | 7.4±0.3 | 127.9±0.3 | 8.6±0.2 |
| 3 | Control | 8.3±0.7 | 149.0±0.1 | 10.0±0.6 |

Thus, animals tolerate adequately the drug when the drug is multiply applied (within 10 days with break at the 3 and 6 days) to the male dogs of different age groups at the therapeutic dose as well as at the dose three times greater than the therapeutic dose, and the drug has no adverse effect on the organism's physiological condition and hematological values.

Example 4. (Study of the tolerance of provided drug in male cats)

Materials and methods

Study regarding tolerance of the drug prepared according to the example 16 was conducted in 9 apparently healthy half-breed male cats at the age of 15 months to 3.5 years weighing 3.5-5.0 kg. The animals were arranged into 3 groups, three individuals in each group:

   1st group - animals received 1 ml of the drug;

   2nd group - animals received 3 ml of the drug;

   3rd group - control.

Drug was applied onto skin in the area of back between the scapulas. Animals were treated daily for 8 days with break at the 3 and 6 days. Distilled water was administered to individuals of the third group.

Clinical observations were performed a day before application of the drug, then 1, 6, 12 hours after and 1, 2, 5 days after the first application of the drug. Assessment of the animals included examination, measurement of body temperature, measuring of heart rate and respiration rate, as well as study of hematological and blood chemistry values.

Results

No abnormalities in terms of the physiological condition of the animals have been detected, appetite preserved, the body temperature ranged from 38.5 to 39.0°C, the heart rate was within the range 100-110 heart beating per minute (the normal range is 100-125), the respiration rate was 20-25 (the normal range is 20-30) resp. mov./min., the overall condition of the animals in the experimental groups was not different from that of the control ones.

Use of the drug did not cause changes in the hematological and blood chemistry values of the animals (tables 3, 4).

Table 3

| № | Group | Erythrocytes, $10^{12}$/l | Hemoglobin, g/l | Leucocytes, $10^9$/l |
|---|---|---|---|---|
| | Effect of the experimental drug on the morphological blood composition of male cats | | | |
| 1 | Treatment with the drug at the therapeutic dose | 6.8±0.3 | 96.2±10.8 | 10.8±0.2 |
| 2 | Treatment with the drug at the dose three times greater than the therapeutic dose | 6.5±0.2 | 88.4±9.4 | 9.2±0.4 |
| 3 | Control | 8.0±0.1 | 98.8±7.8 | 9.7±0.1 |

Table 4

| Name of test | 1st group (therapeutic dose) | 2nd group (the dose three times greater than the therapeutic dose) | 3rd group (control) |
|---|---|---|---|
| Effect of the experimental drug on the blood chemistry values of male cats | | | |
| Total protein, g/l | 56.5±6.2 | 58.8±4.5 | 55.4±3.7 |
| Albumin, g/l | 28.8±2.2 | 27.4±0.9 | 26.5±1.2 |
| Alkaline phosphatase, units/l | 85.2±8.8 | 80.4±4.4 | 71.2±5.6 |
| Bilirubin, mmol/l | 8.8±0.4 | 7.9±0.7 | 3.8±0.1 |
| AST, units/l | 29.6±1.4 | 28.4±1.8 | 26.7±1.2 |
| ALT, units/l | 49.4±4.7 | 52.8±3.4 | 50.6±3.6 |
| Glucose, mmol/l | 3.6±0.1 | 5.4±0.7 | 3.5±0.2 |

Conclusion: male cats tolerated adequately the experimental drug when the drug is multiply applied at the therapeutic dose as well as at the dose 3 times greater than the therapeutic dose.

Example 5. (Subchronic toxicity study in rats)

Materials and methods

Subchronic toxicity study was conducted on 15 outbred apparently healthy male rats. The animals were arranged into three groups, 5 individuals in each group. Effect of the drug on the animals' organism was studied for 15 days at the doses of 1/100 and 1/1000 of LD50. Drug prepared according to the example 16 was applied daily cutaneously to the back area to the 1st group at the dose of 0.07 g/kg and to the 2nd group at the dose of 0.007 g/kg. No drugs were applied to the third group. During the study, haematological and blood chemistry values of the animals were analyzed at start of the experiment and 5 days after the last application of the drug, as well as macro- and microscopic examination of the internal organs was performed (see tables 5, 6).

Results

Table 5

| Tests | Units of measurement | Group of animals | | |
|---|---|---|---|---|
| Haematological and blood chemistry values of rats at start of the experiment and after the experiment | | | | |
| | | 1 (0.07 g/kg) | 2 (0.007 g/kg) | 3 (control) |
| At start of the experiment | | | | |

(continued)

| Haematological and blood chemistry values of rats at start of the experiment and after the experiment | | | | |
|---|---|---|---|---|
| Tests | Units of measurement | Group of animals | | |
| | | 1 (0.07 g/kg) | 2 (0.007 g/kg) | 3 (control) |
| Erythrocytes | $10^{12}$/l | 8.0±0.3 | 7.7±0.5 | 8.0±0.7 |
| Hemoglobin | g/l | 13.8±0.2 | 14.4±0.3 | 14.0±0.4 |
| Leucocytes | $10^9$/l | 5.7±0.6 | 5.9±0.2 | 6.1±0.4 |
| Lymphocytes | % | 67.5±2.1 | 72.4±1.7 | 71.7±0.9 |
| Total protein | g/l | 55.0±0.5 | 56.2±0.7 | 56.1±0.3 |
| Glucose | mmol/l | 5.7±1.1 | 5.5±1.7 | 6.0±0.9 |
| Urea | mmol/l | 4.0±0.2 | 4.5±0.1 | 4.8±0.3 |
| Creatinine | mmol/l | 0.3±0.03 | 0.4±0.02 | 0.5±0.04 |
| Alkaline phosphatase | units/l | 34.8±1.6 | 34.8±1.6 | 28.8±1.2 |
| 5 days after the last application of the drug | | | | |
| Erythrocytes | $10^{12}$/l | 7.7±0.2 | 7.5±0.7 | 8.0±0.3 |
| Hemoglobin | g/l | 12.0±0.7 | 12.8±0.9 | 13.6±0.9 |
| Leucocytes | $10^9$/l | 6.4±0.3 | 6.2±0.2 | 5.8±0.7 |
| Lymphocytes | % | 64.6±4.9 | 66.4±7.7 | 69.2±5.3 |
| Total protein | g/l | 50.8±1.7 | 52.2±2.3 | 54.4±2.9 |
| Glucose | mmol/l | 6.8±0.4 | 6.2±0.2 | 6.0±0.9 |
| Urea | mmol/l | 4.0±0.2 | 4.1±0.3 | 3.9±0.1 |
| Creatinine | mmol/l | 0.4±0.02 | 0.6±0.01 | 0.4±0.03 |
| Alkaline phosphatase | units/l | 34.8±5.6 | 36.2±7.5 | 30.4±4.9 |

Haematological and blood chemistry values of rats in group 1 and 2 were at the level of the parameters in the control (group 3) and corresponded to the physiological norm for animals of this sex and age.

Macro- and microscopic examinations of internal organs of rats demonstrated that serous membranes are smooth, shiny, respiratory tracts are free. Heart, liver, kidneys, spleen, organs of the gastrointestinal tract, pancreas, adrenal glands, thymus, testes have usual consistency, colour, size. Liver of all the rats has normal histostructure, one rat of the 1st group had a moderate focal granular degeneration of hepatocytes. Kidneys: in all cases the hyperaemia of the capillaries was observed, especially in the glomeruli, as well as in the interstitium, the epithelium of the straight and convoluted tubules is not changed. Heart: cardiomyocytes are of normal appearance, adequate vascularization. Lungs: bronchial tree within normal limits. Spleen: an increase in the proportion of red pulp, the reaction of follicles in the form of diffuse boundaries, without centrum lucidum. Pancreas: in all cases, lobules are separated by adipose tissue, in the parenchyma - normal islets and parenchyma. Adrenal glands: normal ratios and plethora of layers. Thymus: large lobules with merging centrum lucidum, moderate plethora. Testes: both in control and in experiment contained mature normal spermatozoa in the epididymis. Small intestine: in all cases corresponded to the age-related histostructure.

Mass coefficients of the internal organs of rats are presented in table 6.

Table 6

| Mass coefficients of internal organs of rats after 15 day application of the experimental drug | | | | | | | |
|---|---|---|---|---|---|---|---|
| Experimental group | Heart | Lungs | Liver | Spleen | Kidneys | Thymus | Testes |
| 1 (0.07 g/kg) | 3.1±0.1 | 6.6±0.7 | 33.0±1.0 | 3.0±0.4 | 6.0±0.2 | 1.0±0.1 | 7.4±0.4 |
| 2 (0.007 g/kg) | 3.7±0.3 | 6.6±0.6 | 34.4±1.6 | 3.3±0.4 | 6.0±0.5 | 1.1±0.3 | 8.4±1.0 |
| 3 (control) | 3.3±0.2 | 6.5±0.3 | 34.5±2.0 | 3.2±0.2 | 6.1±0.2 | 1.1±0.1 | 7.7±0.5 |

Conclusion: conducted studies demonstrated that prolonged use of the experimental drug at the doses exceeding the therapeutic doses does not cause any changes in haematological and blood chemistry values of laboratory animals and does not adversely affect the morphology of the internal organs.

Example 6. (Study regarding the provided drug controlling sexual behaviour in male cats)

Materials and methods

Tests of sexual behaviour controlling drug prepared according to the example 16 were carried out in 4 domestic outbred apparently healthy male cats living in different hosts between May 2013 and November 2014 (see table 7).

Table 7

| Name | Age, years | Weight, kg | Test start date | Duration of effect, months |
|---|---|---|---|---|
| Pirat | 10 | 4 | May 2013 | 3 |
| Markiz | 11 | 4.5 | September 2013 | 6 |
| Botsman | 8 | 3.5 | March 2014 | 2 |
| Bars | 5 | 4 | November 2014 | 3 |

The drug was applied to the animals on the second day of the onset of rutting at the evening time in the amount of 1 ml per back between the scapulas.

Results

After application of the drug the hosts of male cats noted disappearance of signs of rutting, animals calming, reducing aggressiveness, reducing the manifestations of vocalization.
In one male cat, rutting resumed 2 months after the drug was used, in one cat - after 6 months, in two - after 3 months.
No side effects of the drug were observed.
Conclusion: the experimental drug demonstrated high efficacy as a regulator of sexual behaviour for a period of 2 to 6 months in male cats.

Example 7. (Study regarding the provided drug controlling sexual behaviour in male cats)

Materials and methods

Tests for determination the efficacy of using the drug, prepared according to the example 16, in order to reduce sexual activity and correct undesirable behaviour (increased aggressiveness and vocalization, seeking a sexual partner, territorial marking with urine) were carried out at the premises of the shelter for homeless animals: Municipal Non-Governmental Charitable Foundation "Zoozashita Plus", Serpukhov District, Moscow Region, during the period from February to April 2014 on 10 apparently healthy male cats at the age of 12 months to 3 years weighing 3.3-4.8 kg.
The animals were arranged into two groups: experimental and control, 5 individuals in each group. The drug was applied to the male cats of the experimental group once during the evening examination by drip application onto dry intact skin in the area of back between the scapulas at the dose 1 dropper pipette (1 ml) per animal. No drugs were applied to the animals in the control group. The male cats of the experimental and control group were examined once a day.

Results

In the absence of proper effect within 24 hours, the drug was applied to two male cats of the experimental group once more at the same dose. No adverse effects and complications were observed in male cats. Signs of sexual arousal and related behaviour therewith were not observed in experimental animals during the entire follow-up period (67 days), the effect occurred on day 1-2.
Conclusion: the experimental drug demonstrated high efficacy as a regulator of sexual behaviour in male cats. The effect developed rapidly in 1-2 days and maintained for 67 days of observation.

Example 8. (Study regarding the provided drug controlling sexual behaviour in male cats)

Materials and methods

Tests for determination the therapeutic efficacy of the drug prepared according to the example 16 were carried out between January and February 2015 at LLC "Kaliningrad Regional Center for Veterinary Medicine" in 14 apparently healthy male cats at the age of 10 months to 4 years weighing 2.5-4.0 kg.

The animals were arranged into two groups: experimental and control, 7 individuals in each group. In the period of increased sexual activity on day 2-3 of onset of the signs, during the evening clinic appointment the test agent was applied to the male cats of the experimental group once by drip application onto dry intact skin in the area of the back between the scapulas or in the neck region at the base of skull at the dose of 1 ml (20 drops) per animal. No drugs were applied to the animals in the control group and they continued to "sleep around". The male cats of the experimental and control group were examined in clinic once a week.

Results

In the absence of proper effect within 24 hours, the drug was applied to two male cats of the experimental group once more at the same dose. While using the drug no side effects and complications in male cats were observed. Sexual activity in 5 male cats of the experimental group ceased on the 2nd day, in 2 male cats - on the 3rd day. Over 30 days of the study, 4 male cats of the control group had sexual activity recorded three times, in 3 male cats - twice. All the male cats of the experimental group had no sexual activity over 30 days of the experiment.

Conclusion: the experimental drug demonstrated high efficacy as a regulator of sexual behaviour in male cats. The effect developed in 2-3 days and maintained for 30 days of observation.

Example 9. (Study regarding the provided drug controlling sexual behaviour in male cats)

Materials and methods

Tests for determination the therapeutic efficacy of the drug prepared according to the example 19 were carried out between January and March 2015 at LLC "Kaliningrad Regional Center for Veterinary Medicine" in 14 apparently healthy male cats at the age of 11 months to 4 years weighing 2.5-4.5 kg.

The animals were arranged into two groups: experimental and control, 7 individuals in each group. In the period of increased sexual activity, the test drug was applied to the male cats of the experimental group once by drip application onto dry intact skin in the area of the back between the scapulas or in the neck region at the base of skull at the dose of 1 ml (20-25 drops) per animal. Male cats were kept in housing conditions. No drugs were applied to the animals in the control group and they continued to "sleep around". The male cats of the experimental and control group were examined in clinic once a week.

Results

In the absence of proper effect within 24 hours, the drug was applied to three male cats of the experimental group once more at the same dose. While using the drug no side effects and complications in animals were observed. Sexual activity in 4 male cats of the experimental group ceased on the third day, in 3 male cats - on the fourth day. Over 40 days of the study, the sexual activity of male cats from the control group did not stop, animals were "marking" the territory. All the male cats of the experimental group had no sexual activity over 28 days. On the 33rd day 2 male cats marked the territory, on the 35th day 2 male cats "slept around", periodically shouted and worried.

The remaining animals of the experimental group became restless, 2 male cats on the 38th day of the experiment marked the territory, became aggressive.

Conclusion: the experimental drug demonstrated high efficacy as a regulator of sexual behaviour in male cats. The effect developed in 3-4 days and maintained for at least 28 days of observation.

Example 10. (Study regarding the provided drug controlling sexual behaviour in male dogs)

Materials and methods

Tests to determine the efficacy of using the drug, prepared according to the example 16, in order to correct sexual behaviour were carried out at the premises of the shelter for homeless animals: Municipal Non-Governmental Charitable Foundation "Zoozashita Plus", Serpukhov District, Moscow Region, during the period from February to May 2014 on 12 apparently healthy male dogs at the age of 1.5 to 5 years weighing 7-35 kg. The animals were kept at the premises of the shelter.

The animals were arranged into two groups: experimental and control, 6 individuals in each group. The drug was applied to the male dogs of the experimental group once during the evening examination by drip application onto dry intact skin in the area of back between the scapulas at the dose 2 ml per animal weighing of 5 to 15 kg and 4 ml per animal weighing of 15 to 35 kg. No drugs were applied to the animals in the control group. The male dogs of the experimental and control group were examined once a day.

Results

In the absence of proper effect within 24 hours, the drug was applied to two animals of the experimental group once more at the same dose. While using the drug no side effects and complications in male dogs were observed. Signs of reducing aggressiveness and sexual activity in dogs of the experimental group ceased on day 2-3 after the application of the drug. The dogs of the control group behaved without changing behaviour. No significant undesirable behaviour was observed in the animals of the experimental group after 76 days after application of the drug.

Conclusion: the experimental drug demonstrated high efficacy as a regulator of sexual behaviour in male dogs. The effect developed in 2-3 days and maintained for 76 days of observation.

Example 11. (Study regarding the provided drug controlling sexual behaviour in male dogs)

Materials and methods

Tests for determination the therapeutic efficacy of the drug prepared according to the example 16 were carried out between January and February 2015 at LLC "Kaliningrad Regional Center for Veterinary Medicine" in 14 apparently healthy male dogs of different breeds weighing 7-30 kg. All animals were at the stage of rutting.

The animals were arranged into two groups: experimental and control, 7 individuals in each group. Test agent was applied to the male dogs of the experimental group once during the evening clinic appointment by drip application onto dry intact skin in the neck region at the base of skull at the doses by weight of the dog: 5 to 15 kg - 2 ml; 15 to 35 kg - 4 ml. The male dogs of the experimental and control group were examined in clinic once a week.

Results

In the absence of proper effect within 24 hours, the drug was applied to two dogs of the experimental group once more at the same dose. Restless behaviour of the animals in the experimental group disappeared 48-72 hours after the drug was given and did not resume within 30 days (observation period).

Conclusion: the experimental drug demonstrated high efficacy as a regulator of sexual behaviour in male dogs. The effect developed in 2-3 days and maintained for 30 days of observation.

Example 12. (Study regarding the provided drug controlling sexual behaviour in male dogs)

Materials and methods

Tests for determination the therapeutic efficacy of the drug prepared according to the example 19 were carried out during the period between January and March 2015 at LLC "Kaliningrad Regional Center for Veterinary Medicine" in 14 apparently healthy male dogs of different breeds weighing 7-30 kg. All animals were at the stage of rutting.

The animals were arranged into two groups: experimental and control, 7 individuals in each group. Test agent was applied to the male dogs of the experimental group once during the evening clinic appointment by drip application onto dry intact skin in the neck region at the base of skull at the doses by weight of the dog: 5 to 15 kg - 2 ml; 15 to 35 kg - 4 ml. Thereafter, the lady dogs at the stage of estrus were brought to the male dogs and the reaction of the experimental animals to them was evaluated. The male dogs of the experimental and control group were examined in clinic once a week.

Results

In the absence of proper effect within 24 hours, the drug was applied to two dogs of the experimental group once more at the same dose. Restless behaviour of the animals from the experimental group disappeared on 2-3 day after using the drug and did not resume within 30 days (observation period), experimental animals reacted calmly to the dogs in heat. While the male dogs of the control group were in the phase of sexual activity during the whole experiment and actively reacted to the dogs in heat. The sexual activity in 5 male dogs of the experimental group was resumed completely after 30 days after application of the drug. In two dogs treated once more restlessness returned on day 36.

Conclusion: the experimental drug demonstrated high efficacy as a regulator of sexual behaviour in male dogs. The effect developed in 2-3 days and maintained for at least 30 days of observation.

Example 13. (Study regarding the provided drug controlling sexual behaviour in male cats and male dogs)

Materials and methods

Tests for efficacy of the drug, prepared according to the example 16, in order to reduce sexual activity and correct behaviour in male cats and dogs were conducted at the premises of Dzerzhinsky Station of Animals Disease Control during the period from April to July 2014.

There were 8 male cats and 5 male dogs of different breeds at the age of 1.5 to 6 years with signs of undesirable sexual behaviour (increased aggressiveness and vocalization, seeking sexual partner, territorial marking with urine, etc.) under observation. All the animals were apparently healthy. Observation was conducted in the clinic and in conditions of domestic maintenance of animals with the participation of their owners. Drug was used individually once cutaneously in the area of neck and back of animals. The dose for the male cats was 1 ml per individual, for the male dogs - 2 ml per animal weighing of 5 to 15 kg and 4 ml per animal weighing of 15 to 35 kg.

Results

Observation of the condition of animals did not reveal any apparent deviations in the behaviour and physiological status of the animals. Reduction of sexual arousal occurred in male dogs on day 1-3 after giving the drug. A stable sign of "calming" was observed on day 1-2 in male cats. The period of "calming" varied from 3 weeks to 3 months (observation period).

Conclusion: the experimental drug demonstrated high efficacy as a regulator of sexual behaviour in male cats and male dogs. The effect developed for 1-3 days and maintained during 3 weeks to 3 months of observation.

Example 14. (Study regarding the provided drug controlling sexual behaviour in male cats and male dogs)

Materials and methods

Tests of the drug, prepared according to the example 16, intended to reduce sexual activity and to correct behaviour in male cats and male dogs were conducted at the premises of The Main Department of Veterinary Medicine of the Moscow Region - Balashikha Station of Animals Disease Control during the period from March to June 2014. There were 10 male cats and 6 male dogs (mature, fertile, apparently healthy) with signs of undesirable sexual behaviour (increased aggressiveness and vocalization, seeking a sexual partner, territorial marking with urine, etc.) under observation. The drug was applied to the animals once during the evening time cutaneously, based on the body weight of the animals at the doses:

- 1 ml per animal to male cats regardless of weight;

- to male dogs weighing less than 5 kg - 1 ml, 5 to 15 kg - 2 ml; 15 to 35 kg - 4 ml.

Results

As signs of undesirable behaviour maintained, so repeated application of the drug after 24 hours was required for 3 male cats and 2 male dogs. Suppression of negative behaviour in the face of sexual arousal in male cats and male dogs completely occurred within the first 2-3 days.

No side effects and complications in animals in the process of observation were revealed. The resumption of undesirable behaviour was not observed during the observation period (3.5 months).

Conclusion: the experimental drug demonstrated high efficacy as a regulator of sexual behaviour in male cats and male dogs. The effect developed for 2-3 days and maintained during 3.5 months of observations.

Example 15. (Study of suppression of sexual arousal signs by the provided drug in male cats and male dogs)

Materials and methods

Tests for determination the efficacy of using the drug, prepared according to the example 16, for controlling sexual behaviour were carried out at the premises of State-Financed Institution of Pachelma Regional Station of Animals Disease Control, Penza Region, during the period from February to May 2014 in 6 apparently healthy male cats and 8 apparently healthy male dogs.

The drug was applied to the male cats once cutaneously in the area of neck and back at the dose 1 ml per animal regardless of body weight. The drug was applied to the male dogs once cutaneously in the area of neck and back at the dose to the animals weighing less than 5 kg - 1 ml, 5 to 15 kg - 2 ml; 15 to 35 kg - 4 ml. Control of the effect of the drug was carried out every week in owners' words of animals.

Results

The resting stage developed in experimental animals for 12-72 hours. The efficacy of the drug in male cats and male dogs while correcting the behaviour of animals in the face of sexual arousal was 100%. No side effects were reported.

Conclusion: the experimental drug demonstrated 100% efficacy in suppressing signs of undesirable behaviour of animals in the face of sexual arousal. The effect developed over 12-72 hours.

Example 16. (preparation)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of D-panthenol and 5 g of N-methyl-N-(1,2$R$,3$R$,4$R$,5$S$-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.32 by adding stearic acid with the assistance by an ion meter with input impedance of > 2 GOhm. The beaker is wrapped with nontransparent foil. 10 g of proroxan base, 100 g of melatonin and 10 g of 6-chloro-1$\beta$,2$\beta$-dihydro-17-hydroxy-3'H-cyclopropa[1,2]pregna-1,4,6-triene-3,20-dione acetate (cyproterone acetate) are added to the prepared solution. It is being agitated while heating to 40°C until complete dissolution of the components. pH value is adjusted to the value of 4.30 by adding stearic acid. The total amount of stearic acid used for pH adjustment was 11 g. When cooled to room temperature, the solution becomes cloudy. 993 g of transdermal composition with the following parameters are prepared:

1. Description - floating liquid cloudy yellow mass;

2. Density - 1.10 g/cm$^3$;

3. pH 4.31;

4. Content of cyproterone acetate is 10.93 mg/cm3;

5. Content of melatonin is 110.17 mg/cm3;

6. Content of proroxan is 10.78 mg/cm$^3$;

7. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.11 g/cm$^3$;

3. pH 4.29;

4. Content of cyproterone acetate is 10.48 mg/cm$^3$;

5. Content of melatonin is 109.83 mg/cm$^3$;

6. Content of proroxan is 10.17 mg/cm$^3$;

7. Viscosity - 4.5 cPs.

Thus the drug is stable when stored under natural conditions in polymer pipettes for two years.

Example 17. (proroxan and butyroxan)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 1 g of 3-(octadecyloxy)-1,2-propanediol, 5 g *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.32 by adding stearic acid with the assistance by an ion meter with input impedance of > 2 GOhm. The beaker is wrapped with nontransparent foil. 5 g of proroxan base and 5 g of butyroxan base, 100 g of melatonin and 10 g of cyproterone acetate are added to the prepared solution. It is being agitated while heating to 40°C until complete dissolution of the components. pH value is adjusted to the value of 4.25 by adding stearic acid. The total amount of stearic acid used for pH adjustment was 11 g. When cooled to room temperature, the solution becomes cloudy. 993 g of transdermal composition with the following parameters are prepared:

1. Description - floating liquid cloudy yellow mass;

2. Density - 1.10 g/cm$^3$;

3. pH 4.27;

4. Content of cyproterone acetate is 10.93 mg/cm3;

5. Content of melatonin is 110.17 mg/cm$^3$;

6. Content of proroxan is 5.43 mg/cm3;

7. Content of butyroxan is 5.36 mg/cm3;

8. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.11 g/cm$^3$;

3. pH 4.29;

4. Content of cyproterone acetate is 10.48 mg/cm$^3$;

5. Content of melatonin is 109.83 mg/cm$^3$;

6. Content of proroxan is 4.89 mg/cm$^3$;

7. Content of butyroxan is 5.14 mg/cm$^3$;

8. Viscosity - 4.5 cPs.

Thus the drug is stable when stored under natural conditions in polymer pipettes for two years.

Example 18. (butyroxan + tartaric acid)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of D-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.32 by adding tartaric acid with the assistance by an ion meter with input impedance of > 2 GOhm. The beaker is wrapped with nontransparent foil. 10 g of butyroxan base, 100 g of melatonin and 10 g of cyproterone acetate are added to the prepared solution. It is being agitated while heating to 40°C until complete dissolution of the components. pH value is adjusted to the value of 4.25 by adding stearic acid. The total amount of stearic acid used for pH adjustment was 10 g. When cooled to room temperature, the solution becomes cloudy. 992 g of transdermal composition with the following parameters are prepared:

1. Description - floating liquid cloudy yellow mass;

2. Density - 1.10 g/cm$^3$;

3. pH 4.27;

4. Content of cyproterone acetate is 10.93 mg/cm$^3$;

5. Content of melatonin is 110.17 mg/cm$^3$;

6. Content of butyroxan is 11.36 mg/cm$^3$;

7. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.11 g/cm$^3$;

3. pH 4.29;

4. Content of cyproterone acetate is 10.48 mg/cm$^3$;

5. Content of melatonin is 109.83 mg/cm$^3$;

6. Content of butyroxan is 11.14 mg/cm$^3$;

7. Viscosity - 4.5 cPs.

Thus the drug is stable when stored under natural conditions in polymer pipettes for two years.

Example 19. (minimum content of cyproterone)

395 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 10 g of proroxan base, 100 g of melatonin and 5 g of cyproterone acetate are added to the prepared solution. It is being agitated while heating to 40°C until complete dissolution of the components. pH value is adjusted to the value of 4.34 by adding stearic acid. The total amount of stearic acid used for pH adjustment was 11 g. When cooled to room temperature, the solution becomes cloudy. 992 g of transdermal composition with the following parameters are prepared:

1. Description - floating liquid cloudy yellow mass;

2. Density - 1.12 g/cm$^3$;

3. pH 4.32;

4. Content of cyproterone acetate is 5.51 mg/cm$^3$;

5. Content of melatonin is 113.15 mg/cm$^3$;

6. Content of proroxan is 11.16 mg/cm$^3$;

7. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.11 g/cm$^3$;

3. pH 4.34;

4. Content of cyproterone acetate is 5.32 mg/cm$^3$;

5. Content of melatonin is 112.91 mg/cm$^3$;

6. Content of proroxan is 10.94 mg/cm$^3$;

7. Viscosity - 4.5 cPs.

Thus the drug is stable when stored under natural conditions in polymer pipettes for two years.

Example 20. (maximum content of cyproterone)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 1 g of 3-(octadecyloxy)-1,2-propanediol, 5 g of D-panthenol and 5 g of N-methyl-N-(1,2R,3R,4R,5S-pentahydroxyhexyl)-octadecanamide are added into a 2 L beaker fitted with an agitator. The mass is being agitated while heating to 60°C until complete dissolution of the components, then cooled to room temperature. pH value is adjusted to 4.25 by adding stearic acid with the assistance by an ion meter with input impedance of > 2 GOhm. The beaker is wrapped with nontransparent foil. 10 g of proroxan base, 100 g of melatonin and 18.2 g of cyproterone acetate was added to the prepared solution. It is being agitated while heating to 40°C until complete dissolution of the components. pH value is adjusted to the value of 4.30 by adding stearic acid. The total amount of stearic acid used for pH adjustment was 11 g. When cooled to room temperature, the solution becomes cloudy. 1001.2 g of transdermal composition with the following parameters are prepared:

1. Description - floating liquid cloudy yellow mass;

2. Density - 1.10 g/cm$^3$;

3. pH 4.27;

4. Content of cyproterone acetate is 19.96 mg/cm$^3$;

5. Content of melatonin is 109.92 mg/cm3;

6. Content of proroxan is 10.87 mg/cm3;

7. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.09 g/cm$^3$;

3. pH 4.28;

4. Content of cyproterone acetate is 19.22 mg/cm$^3$;

5. Content of melatonin is 109.17 mg/cm3;

6. Content of proroxan is 10.27 mg/cm3;

7. Viscosity - 4.5 cPs.

Thus the drug is stable when stored under natural conditions in polymer pipettes for two years.

Example 21. (average content of melatonin, pyruvic + citric acid)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value was adjusted to 4.27 by adding pyruvic acid with the assistance by an ion meter with input impedance of > 2 GOhm. The beaker is wrapped with nontransparent foil. 10 g of proroxan base, 150 g of melatonin and 10 g of cyproterone acetate are added to the prepared solution. It is being agitated while heating to 40°C until complete dissolution of the components. pH value is adjusted by adding citric acid to the value of 4.28. The amount of pyruvic acid used for pH adjustment was 5 g, and citric acid - 4 g. When cooled to room temperature, the solution becomes cloudy. 1041 g of transdermal composition with the following parameters are prepared:

1. Description - floating liquid cloudy yellow mass;

2. Density - 1.15 g/cm$^3$;

3. pH 4.31;

4. Content of cyproterone acetate is 10.81 mg/cm3;

5. Content of melatonin is 164.13 mg/cm$^3$;

6. Content of proroxan is 10.43 mg/cm3;

7. Viscosity - 4.5 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.13 g/cm$^3$;

3. pH 4.33;

4. Content of cyproterone acetate is 10.35 mg/cm3;

5. Content of melatonin is 162.61 mg/cm3;

6. Content of proroxan is 10.17 mg/cm3;

7. Viscosity - 4.5 cPs.

Thus the drug is stable when stored under natural conditions in polymer pipettes for two years.

Example 22. (maximum content of melatonin)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.25 by adding stearic acid with the assistance by an ion meter with input impedance of > 2 GOhm. The beaker is wrapped with nontransparent foil. 10 g of proroxan base, 185 g of melatonin and 10 g of cyproterone acetate are added to the prepared solution. It is being agitated while heating to 40°C until complete dissolution of the components. pH value is adjusted to the value of 4.29 by adding stearic acid. The total amount of stearic acid used for pH adjustment was 11 g. When cooled to room temperature, the solution becomes cloudy. 1078 g of transdermal composition with the following parameters are prepared:

1. Description - floating liquid cloudy yellow mass;

2. Density - 1.17 g/cm$^3$;

3. pH 4.32;

4. Content of cyproterone acetate is 10.64 mg/cm$^3$;

5. Content of melatonin is 200.54 mg/cm$^3$;

6. Content of proroxan is 10.57 mg/cm$^3$;

7. Viscosity - 4.5 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.14 g/cm$^3$;

3. pH 4.31;

4. Content of cyproterone acetate is 10.09 mg/cm$^3$;

5. Content of melatonin is 199.04 mg/cm$^3$;

6. Content of proroxan is 10.14 mg/cm$^3$;

7. Viscosity - 4.5 cPs.

Thus the drug is stable when stored under natural conditions in polymer pipettes for two years.

Example 23. (average content of proroxan)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 15 g of proroxan base, 100 g of melatonin and 10 g of cyproterone acetate are added to the prepared solution. It is being agitated while heating to 40°C until complete dissolution of the components. pH value is adjusted to the value of 4.29 by adding stearic acid. The total amount of stearic acid used for pH adjustment was 11 g. When cooled to room temperature, the solution becomes cloudy. 998 g of transdermal composition with the following parameters are prepared:

1. Description - floating liquid cloudy yellow mass;

2. Density - 1.09 g/cm$^3$;

3. pH 4.30;

4. Content of cyproterone acetate is 10.57 mg/cm3;

5. Content of melatonin is 108.99 mg/cm3;

6. Content of proroxan is 16.02 mg/cm3;

7. Viscosity - 4.5 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.10 g/cm$^3$;

3. pH 4.32;

4. Content of cyproterone acetate is 10.12 mg/cm3;

5. Content of melatonin is 107.21 mg/cm3;

6. Content of proroxan is 15.48 mg/cm$^3$;

7. Viscosity - 4.5 cPs.

Thus the drug is stable when stored under natural conditions in polymer pipettes for two years.

Example 24. (maximum content of proroxan)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 18 g of proroxan base, 100 g of melatonin and 10 g of cyproterone acetate are added to the prepared solution. It is being agitated while heating to 40°C until complete dissolution of the components. pH value is adjusted to the value of 4.29 by adding stearic acid. The total amount of stearic acid used for pH adjustment was 11 g. When cooled to room temperature, the solution becomes cloudy. 1001 g of transdermal composition with the following parameters are prepared:

1. Description - floating liquid cloudy yellow mass;

2. Density - 1.10 g/cm$^3$;

3. pH 4.31;

4. Content of cyproterone acetate is 10.75 mg/cm3;

5. Content of melatonin is 109.37 mg/cm3;

6. Content of proroxan is 19.54 mg/cm3;

7. Viscosity - 4.5 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.08 g/cm$^3$;

3. pH 4.27;

4. Content of cyproterone acetate is 10.27 mg/cm3;

5. Content of melatonin is 108.02 mg/cm$^3$;

6. Content of proroxan is 19.28 mg/cm$^3$;

7. Viscosity - 4.5 cPs.

Thus the drug is stable when stored under natural conditions in polymer pipettes for two years.

Example 25. (dienogest)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of D-panthenol and 5 g of N-methyl-N-(1,2$R$,3$R$,4$R$,5$S$-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 10 g of proroxan base, 100 g of melatonin and 10 g of 17$\alpha$-cyanomethyl-17$\beta$-hydroxyestra-4,9-diene-3-one (dienogest) are added to the prepared solution.

It is being agitated while heating to 40°C until complete dissolution of the components. pH value is adjusted to the value of 4.32 by adding stearic acid. The total amount of stearic acid used for pH adjustment was 11 g. When cooled to room temperature, the solution becomes cloudy. 993 g of transdermal composition with the following parameters are prepared:

1. Description - floating liquid cloudy yellow mass;

2. Density - 1.08 g/cm$^3$;

3. pH 4.30;

4. Content of dienogest is 10.64 mg/cm$^3$;

5. Content of melatonin is 108.59 mg/cm$^3$;

6. Content of proroxan is 10.71 mg/cm$^3$;

7. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.10 g/cm$^3$;

3. pH 4.28;

4. Content of dienogest is 10.05 mg/cm3;

5. Content of melatonin is 108.01 mg/cm3;

6. Content of proroxan is 9.92 mg/cm3;

7. Viscosity - 4.5 cPs.

Thus the drug is stable when stored under natural conditions in polymer pipettes for two years.

Example 26. (dienogest and cyproterone acetate)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of *D*-panthenol and 5 g of N-methyl-N-(1,2*R*,3*R*,4*R*,5*S*-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 10 g of proroxan base, 100 g of melatonin, and 5 g of dienogest, and 5 g of cyproterone acetate are added to the prepared solution. It is being agitated while heating to 40°C until complete dissolution of the components. pH value is adjusted to the value of 4.32 by adding stearic acid. The total amount of stearic acid used for pH adjustment was 11 g. When cooled to room temperature, the solution becomes cloudy. 993 g of transdermal composition with the following parameters are prepared:

1. Description - floating liquid cloudy yellow mass;

2. Density - 1.08 g/cm$^3$;

3. pH 4.30;

4. Content of dienogest is 5.58 mg/cm3;

5. Content of cyproterone acetate is 5.41 mg/cm3;

6. Content of melatonin is 108.59 mg/cm3;

7. Content of proroxan is 10.71 mg/cm3;

8. Viscosity - 4.5 cPs.

The drug is packed in polypropylene-polyvinyl chloride pipettes of 1 ml. After 2 years of storage the parameters of the drug are as follows:

1. Description - floating cloudy liquid mass, light-yellow solution;

2. Density - 1.10 g/cm$^3$;

3. pH 4.28;

4. Content of dienogest is 5.15 mg/cm3;

5. Content of cyproterone acetate is 5.02 mg/cm3;

6. Content of melatonin is 108.01 mg/cm$^3$;

7. Content of proroxan is 9.92 mg/cm$^3$;

8. Viscosity - 4.5 cPs.

Thus the drug is stable when stored under natural conditions in polymer pipettes for two years.

Example 27. (drospirenone)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 1 g of 3-(octadecyloxy)-1,2-propanediol, 5 g of D-panthenol and 5 g of N-methyl-N-(1,2R,3R,4R,5S-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.30 by adding stearic acid with the assistance by an ion meter with input impedance of > 2 GOhm. The beaker is wrapped with nontransparent foil. 10 g of proroxan base, 100 g of melatonin and 10 g of (6R,7R,8R,9S,10R,13S,14S,15S,16S,17S)-1,3',4',6,6a,7,8,9,10,11,12,13,14,15,15a,16-hexadecahydro-10,13-dimethylspiro-[17H-dicyclopropa-[6,7: 15,16]cyclopenta[a]phenanthrene-17,2'(5'H)-furan] -3,5 '(2H)dione (drospirenone) are added to the prepared solution. It is being agitated while heating to 40°C until complete dissolution of the components. pH value is adjusted to the value of 4.32 by adding stearic acid. The total amount of stearic acid used for pH adjustment was 11 g. When cooled to room temperature, the solution becomes cloudy. 993 g of transdermal composition with the following parameters are prepared:

1. Description - floating liquid cloudy yellow mass;

2. Density - 1.09 g/cm$^3$;

3. pH 4.30;

4. Content of drospirenone is 10.75 mg/cm$^3$;

5. Content of melatonin is 109.57 mg/cm$^3$;

6. Content of proroxan is 10.67 mg/cm$^3$;

7. Viscosity - 4.5 cPs.

The drug is packed in polypropylene-polyvinyl chloride pipettes of 1 ml. After two years of storage the parameters of the drug are as follows:

1. Description - floating cloudy liquid mass, light-yellow solution;

2. Density - 1.08 g/cm$^3$;

3. pH 4.28;

4. Content of drospirenone is 10.03 mg/cm$^3$;

5. Content of melatonin is 108.86 mg/cm$^3$;

6. Content of proroxan is 10.17 mg/cm$^3$;

7. Viscosity - 4.5 cPs.

Thus the drug is stable when stored under natural conditions in polymer pipettes for two years.

Example 28. (without proroxan)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 1 g of 3-(octadecyloxy)-1,2-propanediol, 5 g of D-panthenol and 5 g of N-methyl-N-(1,2R,3R,4R,5S-pentahydroxyhexyl)-octadecanamide are added into a 2 L beaker fitted with an agitator. The mass is being agitated while heating to 60°C until complete dissolution of the components, and then cooled to room temperature. pH value is adjusted to 4.17 by adding stearic acid with the assistance by an ion meter with input impedance of > 2 GOhm. The beaker is wrapped with nontransparent foil. 100 g of melatonin and 10 g of cyproterone acetate are added to the prepared solution. It is being agitated while heating to 40°C until complete dissolution of the components. pH value is adjusted to the value of 4.45 by adding stearic acid. The total amount of stearic acid used for pH adjustment was 9 g. When cooled to room temperature, the solution becomes cloudy. 981 g of transdermal composition with the following parameters are prepared:

1. Description - floating liquid cloudy yellow mass;

2. Density - 1.08 g/cm$^3$;

3. pH 4.41;

4. Content of cyproterone acetate is 10.14 mg/cm$^3$;

5. Content of melatonin is 115.17 mg/cm$^3$;

6. Viscosity - 4.5 cPs.

The drug is packed in polypropylene-polyvinyl chloride pipettes of 1 ml.

Example 29. (non-selective α-adrenergic blocking agent - tropaphenum)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2R,3R,4R,5S-pentahydroxyhexyl)-octadecanamide and 1 g of 3-(octadecyloxy)-1,2-propanediol are added into a 2 L beaker fitted with an agitator. The mass is being agitated while heating to 60°C until
complete dissolution of the components. It is cooled to room temperature while agitating. pH value is adjusted to 4.37 by adding stearic acid with the assistance by an ion meter with input impedance of > 2 GOhm. The beaker is wrapped with nontransparent foil. 15 g of tropaphenum, 100 g of melatonin and 10 g of cyproterone acetate are added to the prepared solution. The mass is being agitated while heating to 40°C until complete dissolution of the components. pH value is adjusted to the value of 4.30 by adding stearic acid. The total amount of stearic acid used for pH adjustment was 10 g. When cooled to room temperature, the solution becomes cloudy. 997 g of transdermal composition with the following parameters are prepared:

1. Description - floating liquid cloudy yellow mass;

2. Density - 1.12 g/cm$^3$;

3. pH 4.33;

4. Content of cyproterone acetate is 10.96 mg/cm$^3$;

5. Content of melatonin is 132.70 mg/cm$^3$;

6. Content of tropaphenum is 16.38 mg/cm$^3$;

7. Viscosity - 4.6 cPs.

The drug is packed in polypropylene-polyvinyl chloride pipettes of 1 ml.

Example 30. (selective α-adrenergic blocking agent - prazosin)

391 g of 1,2,3-triacetoxypropane, 150 g of N,N-diethyltoluamide, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2R,3R,4R,5S-pentahydroxyhexyl)-octadecanamide and 1 g of 3-(octadecyloxy)-1,2-propanediol are added into a 2 L beaker fitted with an agitator. The mass is being agitated while heating to 60°C until complete dissolution of the components. It is cooled to room temperature while agitating. pH value is adjusted to 4.30 by adding stearic acid with the assistance by an ion meter with input impedance of > 2 GOhm. The beaker is wrapped with nontransparent foil. 2,5 g of prazosin, 100 g of melatonin and 10 g of cyproterone acetate are added to the prepared solution. The mass is being agitated while heating to 40°C until complete dissolution of the components. pH value is adjusted to the value of 4.15 by adding stearic acid. The total amount of stearic acid used for pH adjustment was 7 g. When cooled to room temperature, the solution becomes cloudy. 992 g of transdermal composition with the following parameters are prepared:

1. Description - floating liquid cloudy yellow mass;

2. Density - 1.10 g/cm$^3$;

3. pH 4.11;

4. Content of cyproterone acetate is 11.73 mg/cm$^3$;

5. Content of melatonin is 112.16 mg/cm$^3$;

6. Content of prazosin is 2.39 mg/cm$^3$;

7. Viscosity - 4.6 cPs.

The drug is packed in polypropylene-polyvinyl chloride pipettes of 1 ml.

Example 31. (Study of the drugs' effect: with proroxan, without proroxan, with tropaphenum, with prazosin)

Materials and methods

The effects of the drugs prepared according to examples 16, 28, 29 and 30 were studied on 25 sexually mature apparently healthy male cats at the age of 1.5-7 years arranged into 5 groups, 3 individuals in each group. The cats were fed with a standard diet using dry food, canned goods and sufficient amount of drinking water. The drugs were used once at the dosage of 1 ml per individual by application on intact skin along the spinal cord between the scapulas.

Group 1 received the drug according to the example 16,

group 2 - the drug in accordance with example 28,

group 3 - the drug in accordance with example 29,

group 4 - the drug in accordance with example 30.

No drugs were applied to the control group.

The experimental agents were applied to the animals in the phase of increased sexual activity, as evidenced by restless sexual behaviour (vocalization, interest in the opposite gender, aggressiveness, etc.) and testosterone values in the blood.

In the course of the experiment the content of testosterone in the blood of the experimental animals was analysed.

The content of the hormone was defined using radioimmune chemiluminescent method. Venous blood sampling was performed under fasting conditions in amount of 1-2 ml before administration, 12, 24 hours after transdermal administration of the experimental drugs and subsequently once a week. Blood samples were stored in the refrigerator. Whole blood samples were used for serum preparation.

Results

Table 8

| Content of testosterone in the blood, nmol/l | | | | | | | | |
|---|---|---|---|---|---|---|---|---|
| Group | № | Before | After 1 week | After 1 month | After 2 months | After 3 months | After 4 months | After 5 months |
| Control | 1 | 54.8 | 50.2 | 54.2 | 55.3 | 50.9 | 45.1 | 52.1 |
| | 2 | 72.4 | 75.9 | 70.1 | 80.7 | 68.6 | 57.8 | 50.9 |
| | 3 | 81.5 | 75.3 | 77.5 | 80.1 | 59.7 | 75.4 | 66.9 |
| 1st group | 1 | 59.6 | 11.3 | 10.5 | 11.8 | 15.7 | 10.6 | 14.1 |
| | 2 | 61.4 | 15.7 | 16.5 | 21.0 | 19.9 | 14.8 | 52.8 |
| | 3 | 83.1 | 12.2 | 12.9 | 14.8 | 52.4 | 66.0 | 44.5 |
| 2nd group | 1 | 44.9 | 8.9 | 18.8 | 59.4 | 46.5 | 72.6 | 34.5 |
| | 2 | 47.9 | 12.3 | 15.2 | 49.3 | 59.9 | 56.4 | 25.0 |
| | 3 | 79.2 | 16.1 | 21.7 | 62.9 | 77.1 | 63.0 | 50.4 |
| 3rd group | 1 | 50.3 | 12.7 | 11.6 | 55.4 | 48.7 | 62.5 | 44.4 |
| | 2 | 68.8 | 17.1 | 15.4 | 65.9 | 64.4 | 70.4 | 60.7 |
| | 3 | 48.7 | 21.6 | 12.9 | 50.6 | 46.1 | 36.4 | 40.6 |
| 4th group | 1 | 66.3 | 23.6 | 43.7 | 46.0 | 55.4 | 67.4 | 48.0 |
| | 2 | 47.8 | 15.4 | 41.8 | 52.1 | 54.5 | 46.5 | 50.4 |
| | 3 | 45.3 | 20.2 | 22.1 | 45.7 | 48.3 | 51.9 | 52.0 |

Conclusion: the provided results demonstrate that application of the drug according to the example 16 has a regulatory effect on sexual behaviour, reducing the level of testosterone to the hormone level in male cats in the phase of anestrus for period of up to 5 months (observation period). Application of the drugs according to the examples 28, 29 and 30 also reduces levels of the studied hormones to the basal level however only for a period from 1 week (group 4) to 1 month (group 2-4), after which their concentrations increase to the level corresponding to the period of sexual activity. Application of the drugs according to the examples 28, 29 and 30 is followed by animal apathy and hypotonia.

Example 32. (pH > 6)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 5 g of *D*-panthenol, 5 g of N-methyl-N-(1,2*R*,3*R*,4*R*,5*S*-pentahydroxyhexyl)-octadecanamide and 1 g of 3-(octadecyloxy)-1,2-propanediol are added into a 2 L beaker fitted with an agitator. The mass is being agitated while heating to 60°C until complete dissolution of the components. It is cooled to room temperature while agitating. The beaker is wrapped with nontransparent foil. 10 g of proroxan, 100 g of melatonin and 10 g of cyproterone acetate are added to the prepared solution. The mass was being agitated while heating to 40°C until complete dissolution of the components. With the assistance by an ion meter with input impedance of > 2 GOhm pH value was 7.78. When cooled to room temperature, the solution becomes cloudy. 982 g of transdermal composition with the following parameters are prepared:

1. Description - floating liquid cloudy yellow mass;

2. Density - 1.09 g/cm$^3$;

3. pH 7.80;

4. Content of cyproterone acetate is 10.18 mg/cm$^3$;

5. Content of melatonin is 151.01 mg/cm$^3$;

6. Content of proroxan is 10.33 mg/cm$^3$;

7. Viscosity - 4.6 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.10 g/cm$^3$;

3. pH 7.84;

4. Content of cyproterone acetate is 4.31 mg/cm$^3$;

5. Content of melatonin is 118.38 mg/cm$^3$;

6. Content of proroxan is 7.52 mg/cm$^3$;

7. Viscosity - 4.6 cPs.

Thus, if the pH exceeds 6, the drug is not stable when stored under natural conditions in polymer pipettes for two years.

Example 33. (pH < 4)

391 g of 1,2,3-triacetoxypropane, 150 g of N,N-diethyltoluamide, 200 g of 1,2-isopropylidene glycerol, 100 g of polyoxyethylated $\alpha$-tocopherol, 10 g of polyoxyethylated lanolin, 5 g of D-panthenol, 5 g of N-methyl-N-(1,2R,3R,4R,5S-pentahydroxyhexyl)-octadecanamide and 1 g of 3-(octadecyloxy)-1,2-propanediol are added into a 2 L beaker fitted with an agitator. The mass is being agitated while heating to 60°C until complete dissolution of the components. It is cooled to room temperature while agitating. pH value is adjusted to 3.37 by adding stearic acid with the assistance by an ion meter with input impedance of > 2 GOhm. The beaker is wrapped with nontransparent foil. 10 g of butyroxan, 100 g of melatonin and 10 g of cyproterone acetate are added to the prepared solution. The mass is being agitated while heating to 40°C until complete dissolution of the components. pH value is adjusted by adding trichloroacetic acid to the value of 3.02. The total amount of stearic acid used for pH adjustment was 23 g. When cooled to room temperature, the solution becomes cloudy. 1005 g of transdermal composition with the following parameters are prepared:

1. Description - floating liquid cloudy yellow mass;

2. Density - 1.15 g/cm$^3$;

3. pH 3.35;

4. Content of cyproterone acetate is 11.33 mg/cm$^3$;

5. Content of melatonin is 117.16 mg/cm$^3$;

6. Content of proroxan is 11.79 mg/cm$^3$;

7. Viscosity - 4.6 cPs.

The drug is packed in polypropylene-polyvinyl chloride pipettes of 1 ml. After 2 years of storage the parameters of the drug are as follows:

1. Description - floating cloudy liquid light-yellow mass;

2. Density - 1.12 g/cm$^3$;

3. pH 3.25;

4. Content of cyproterone acetate is 10.94 mg/cm$^3$;

5. Content of melatonin is 115.72 mg/cm$^3$;

6. Content of proroxan is 11.65 mg/cm$^3$;

7. Viscosity - 4.6 cPs.

Thus, if the pH is below 4, the drug is stable when stored under natural conditions in polymer pipettes for two years.

When studies were conducted with animals in the experimental groups a severe persistent reddening of the skin covering in the area of application of the drug has been observed, which led to formation of a 0.3 mm thick skin fold that did not disappear after 20 minutes; the animals exhibit anxiety and attempted to scrape the drug off.

**Claims**

1. A method for controlling sexual behaviour in male animals, comprising administering of melatonin and gestagen, **characterized in that** α-adrenergic blocking agent tropic to the posterior nuclei of the hypothalamus is further administered, wherein the combination of the specified biologically active substances is administered in therapeutic doses during any period of sexual cycle.

2. The method according to claim 1, **characterized in that** proroxan and(or) butyroxan are used as α-adrenergic blocking agent tropic to the posterior nuclei of the hypothalamus.

3. A pharmaceutical composition for controlling sexual behaviour in male animals, which is intended for "Spot-on" transdermal administration, having pH in the range of 4.0 to 6.0 and comprising therapeutic amounts of melatonin, gestagen and α-adrenergic blocking agent tropic to the posterior nuclei of the hypothalamus with the following ratio of the components (weight%):

| | |
|---|---|
| Gestagen | Therapeutic amounts |
| Melatonin | Therapeutic amounts |
| α-Adrenergic blocking agent tropic to the posterior nuclei of the hypothalamus | Therapeutic amounts |
| N-Methyl-N-(1,2*R*,3*R*,4*R*,5*S*-pentahydroxyhexyl)-octadecanamide | 0.5 |
| D-Panthenol | 0.5 |
| Polyoxyethylated α-tocopherol | 9.3-10.2 |
| Polyoxyethylated lanolin | 1 |
| Carboxylic acid | 0.1-1.1 |
| 3-(Octadecyloxy)-1,2-propanediol | 0.1 |
| N,N-Diethyltoluamide | 13.9-15.3 |
| 1,2-Isopropylidene glycerol | 18.6-20.4 |
| 1,2,3-Triacetoxypropane | up to 100 |

4. The pharmaceutical composition according to claim 3, **characterized in that** 6-chloro-1β,2β-dihydro-17-hydroxy-3'H-cyclopropa[1,2]pregna-1,4,6-triene-3,20-dione acetate (cyproterone acetate), or(and) 17α-cyanomethyl-17β-hydroxyestra-4,9-diene-3-one (dienogest), or(and) (6R,7R,8R,9S,10R,13S,14S,15S,16S,17S)-1,3',4',6,6a,7,8,9,10,11,12,13,14,15,15a,16-hexadecahydro-10,13-dimethylspiro-[17H-dicyclopropa-[6,7:15,16]cyclopenta[a]phenanthrene-17,2'(5'H)-furan]-3,5'(2H)dione(drospirenone) are used as gestagen.

**5.** The pharmaceutical composition according to claim 3, **characterized in that** proroxan or(and) butyroxan is used as $\alpha$-adrenergic blocking agent tropic to the posterior nuclei of the hypothalamus.

**EP 3 332 778 A1**

## INTERNATIONAL SEARCH REPORT

| International application No. |
| --- |
| PCT/RU 2016/000498 |

**A. CLASSIFICATION OF SUBJECT MATTER**

A61K 31/00 (2006.01); A61K 31/4045 (2006.01 ); A61K 31/57 (2006.01); A61P 15/00 (2006.01)

According to International Patent Classification (IPC) or to both national classification and IPC

**B. FIELDS SEARCHED**

Minimum documentation searched (classification system followed by classification symbols)

A61K 31/00, 31/4045, 31/57, A61P 15/00

Documentation searched other than minimum documentation to the extent that such documents are included in the fields searched

Electronic data base consulted during the international search (name of data base and, where practicable, search terms used)

PAJ, Esp@cenet, PCT Online, USPTO DB, CIPO (Canada PO), SIPO DB, PubMed, EAPO, PatSearch

**C. DOCUMENTS CONSIDERED TO BE RELEVANT**

| Category* | Citation of document, with indication, where appropriate, of the relevant passages | Relevant to claim No. |
| --- | --- | --- |
| D, Y <br> A | RU 2233586 C1 (ZEINALOV OPXAH AKHMED-OGLY et al.) 10.08.2004, example 2, the claims | 1-2 <br> 3-5 |
| D, Y | EP 0246910 A2 (GENE LINK AUSTRALIA LIMITED et al.) 25.11.1987, the abstract, examples 15, 16 | 1-2 |
| D, Y | MASHKOVSKY M.D. Lekarstvennye sredstva. M., «Novaia volna», 2012, p.262-263 | 1-2 |
| A | TEMURIANTS N. A. et al. Ekzogennyi melatonin vliiaet na polovoe povedenie krys-samtsov, nakhodiashchikhsia v usloviiakh elektromagnitnogo ekranirovaniia. Uchenye zapiski Tavricheskogo natsionalnogo universiteta V. I.Vernadskogo. Seriia "Biologiia, khimiia", Simferopol, 2014, v.27(66), No. 4, p.75-80 | 1-5 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 November 2016 (17.11.2016) | 26 January 2017 (26.01.2017) |

| Name and mailing address of the ISA/ RU | Authorized officer |
| --- | --- |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2163809
- RU 2114621
- EP 0641565 A
- EP 2253228 A
- EP 0246910 A
- RU 2233586

### Non-patent literature cited in the description

- Muzhskaia kontratseptsiia - zavtrashnii den'! [Men's contraception - future!. **SHCHEKINA E.G.** Provisor. 2004
- **MASHKOVSKIJ M.D.** Lekarstvennye sredstva [Medicines. 2012
- **HUNDANOV L. ; GLADKOV A. ; LEVASHOVA V.** Zhit' dolgo i schastlivo. Uchenyh zainteresoval melatonin [Live long and happy. Scientists interested in melatonin. *Medical newsletter,* 1997, vol. 77 (10), 1-3
- **KORHOV V.V. et al.** Postkoital'nye steroidy i nesteroidnye kontraceptivy [Postcoital steroids and nonsteroidal contraceptives. *Pharmacology and toxicology,* 1980, vol. 43 (1), 94-96
- **KORHOV V.V. ; VESHCHILOVA T.P.** Kontraceptivnaja aktivnost' sochetanija steroidov i antiadrenergicheskih preparatov [Contraceptive activity of a combination of steroids and antiadrenergic drugs. *Bull. experim. biol. and med.,* 1975, vol. 79 (4), 77-79
- **KATZUNG B.G.** *Bazisnaia i klinicheskaia farmakologiia [Basic and Clinical Pharmacology,* 2008, vol. 2, 784
- Methodological Recommendations for the Study of the General Toxic Effects of Pharmacological Substances. Ministry of Health of Russia, 2005